# FASCICULE DE BREVET EUROPEEN

(11) **EP 1 728 491 B1**
(45) Date de publication et mention de la délivrance du brevet: **24.02.2016**
(21) Numéro de dépôt: 06356053.6
(22) Date de dépôt: 12.05.2006
(51) Int. Cl.: A61F 2/42, A61F 2/46

(54) **Dispositif chirurgical de traitement du pied plat et kit chirurgical correspondant**
Chirurgische Vorrichtung zum Behandeln von Plattfüssen und entsprechender chirurgischer Teilesatz.
Surgical device for treating flat foot and corresponding surgical kit.

(30) Priorité: 13.05.2005 FR 0504877
(43) Date de publication de la demande: 06.12.2006
(73) Titulaire: Newdeal, 69006 Lyon (FR)
(72) Inventeur: Viladot Perice, Ramon, 08022 Barcelone (ES); Diebold, Patrice François, 54000 Nancy (FR); Dereymaeker, Greta, 3050 Oud-Heverlee (BE); Hintermann, Beat, 4125 Riehen (CH)
(74) Mandataire: Martin, Didier Roland Valéry

(56) Documents cités:
- EP-A- 0 560 249
- US-A- 6 136 032

## Description

La présente invention se rapporte au domaine technique général des dispositifs chirurgicaux utilisés pour le traitement de la pathologie du « *pied plat* », notamment de l'enfant ou de l'adulte.

La présente invention concerne plus particulièrement un dispositif chirurgical de traitement de la pathologie du pied plat comprenant au moins deux composants :
- une enveloppe externe expansible,
- un organe d'expansion, apte à se déplacer au sein de ladite enveloppe externe suivant une direction de compression, ladite enveloppe externe et ledit organe d'expansion étant conformés pour que le déplacement de l'organe d'expansion suivant la direction de compression entraîne l'expansion radiale de ladite enveloppe externe.

Un tel dispositif chirurgical est déscrit dans le document US-6136032.

Le phénomène de pied plat est très souvent dû au défaut d'alignement de l'astragale et du calcanéum, qui provoque une déformation de l'arche plantaire:

Pour remédier à ce type de pathologie, il est connu d'introduire un implant, aussi connu sous l'appellation « *endorthèse »* dans le sinus du tarse. Cet implant joue alors le rôle d'une cale dont la fonction est de rétablir l'alignement entre le calcanéum et l'astragale. Lorsque le réalignement du calcanéum et de l'astragale est satisfaisant, c'est-à-dire en général environ neuf mois après la pose, l'implant est retiré par extraction avec une pince.

Parmi les implants connus, il existe des implant comportant une enveloppe externe en polyéthylène, se présentant sous la forme d'un cylindre percé en son centre d'un tunnel dans lequel on introduit une vis. L'enveloppe externe comporte des entailles permettant son expansion sous l'effet de l'avancée de la vis. En s'expansant, l'implant ouvre le canal du sinus tarsien pour repositionner l'astragale sur le calcanéum.

L'inconvénient majeur de ce type d'implant est de ne pas présenter, dans sa configuration expansée, une forme complémentaire à celle de la cavité du sinus tarsien.

Pour palier cet inconvénient, on a réalisé un autre dispositif chirurgical, formé par trois composants, à savoir:
- un corps central pourvu d'un filetage externe, avec une tête de blocage en translation,
- un cône d'expansion, pourvu d'un filetage interne, apte à coopérer avec le filetage externe du corps central,
- et une couronne externe pouvant être expansée sous l'effet du déplacement du cône d'expansion dans ladite couronne externe lors du vissage du corps central.

De tels dispositifs donnent généralement de bons résultats. En particulier, ces dispositifs peuvent, grâce à leur forme particulière, être bloqués facilement au sein de la cavité du sinus tarsien. Toutefois, ces dispositifs souffrent également de plusieurs inconvénients non négligeables.

En particulier, le système d'assemblage par vissage, qui présente l'avantage d'être réversible et donc de faciliter l'extraction précoce de l'implant en réduisant son diamètre, par dévissage du cône d'expansion, souffre du fait que le cône d'expansion peut se dévisser spontanément, alors que le dispositif est implanté. Dans ce cas, l'efficacité de l'implant est réduite, ce dernier n'étant plus en mesure de jouer son rôle de cale.

Les objets assignés à l'invention visent par conséquent à porter remède aux différents inconvénients énumérés précédemment et à proposer un nouveau dispositif chirurgical de traitement de la pathologie du « *pied plat »* présentant une fiabilité améliorée par rapport aux dispositifs antérieurs.

Un autre objet de l'invention vise à proposer un nouveau dispositif chirurgical qui puisse être facilement introduit puis bloqué en position dans la cavité du sinus tarsien.

Un autre objet de l'invention vise à proposer un nouveau dispositif chirurgical dont la configuration expansée soit particulièrement bien adaptée à la géométrie de la cavité du sinus tarsien.

Les objets assignés à l'invention visent également à proposer un nouveau kit chirurgical de traitement de la pathologie du pied plat permettant d'implanter un dispositif chirurgical dans la cavité du sinus tarsien de façon précise et fiable.

Un autre objet de l'invention vise à proposer un nouveau kit chirurgical de traitement de la pathologie du pied plat qui soit particulièrement facile à manipuler par un chirurgien.

Les objets assignés à l'invention visent également à proposer une nouvelle méthode chirurgicale de traitement de la pathologie du pied plat permettant d'assurer, à l'aide d'un dispositif chirurgical implantable, le réalignement du calcanéum et de l'astragale et ce avec une fiabilité améliorée par rapport aux méthodes de l'art antérieur.

Les objets assignés à l'invention sont atteints à l'aide d'un nouveau dispositif chirurgical de traitement de la pathologie du pied plat comprenant au moins deux composants :
- une enveloppe externe expansible,
- un organe d'expansion, apte à se déplacer au sein de ladite enveloppe externe suivant une direction de compression, ladite enveloppe externe et ledit organe d'expansion étant conformés pour que le déplacement de l'organe d'expansion suivant la direction de compression entraîne l'expansion radiale de ladite enveloppe externe,
caractérisé en ce qu'il comporte des moyens de blocage anti-retour, conformés pour autoriser le déplacement de l'organe d'expansion, selon la direction de compression, vers une position fonctionnelle au sein de l'enveloppe externe et s'opposer au déplacement en sens inverse de l'organe d'expansion une fois ladite position fonctionnelle atteinte, de manière à empêcher l'expulsion de l'organe d'expansion.

Les objets assignés à l'invention sont également atteints à l'aide d'un nouveau kit chirurgical de traitement de la pathologie du pied plat comportant :
- un dispositif chirurgical tel que décrit précédemment, comprenant un moyen de préhension,
- un instrument de pose, apte à être raccordé mécaniquement au moyen de préhension et à exercer un effort de traction sur ce dernier, tout en repoussant l'organe d'expansion selon la direction de compression de telle sorte que ce dernier pénètre progressivement à l'intérieur de l'enveloppe externe en provoquant son expansion.

Les objets assignés à l'invention sont également atteints à l'aide d'une nouvelle méthode chirurgicale de traitement de la pathologie du pied plat comprenant :
- une étape d'implantation, au cours de laquelle on introduit un dispositif chirurgical, comprenant une enveloppe externe expansible, dans le sinus du tarse du pied,
- une étape d'expansion, au cours de laquelle on introduit et on déplace un organe d'expansion dans l'enveloppe externe de manière à provoquer son expansion,
caractérisé en ce qu'elle comprend en outre une étape de blocage, au cours de laquelle on empêche, à l'aide de moyens de blocage anti-retour, l'expulsion de l'organe d'expansion hors de l'enveloppe externe une fois sa position fonctionnelle au sein de ladite enveloppe externe atteinte.

D'autres objets et avantages de l'invention apparaîtront plus en détails à la lecture de la description qui suit, ainsi qu'à l'aide des dessins annexés, donnés à titre purement illustratif et non limitatif, dans lesquels :
- La figure 1 illustre, selon une vue de profil, un dispositif chirurgical de traitement de la pathologie du pied plat conforme à l'invention, dans une position de pré-assemblage précédant son expansion radiale.
- La figure 2 illustre, selon une vue en coupe suivant la ligne A-A illustrée sur la figure 1, le dispositif chirurgical représenté sur la figure 1.
- La figure 3 illustre, selon une vue en coupe, le dispositif chirurgical illustré sur les figures 1 et 2, dans sa position fonctionnelle expansée, pourvu d'un moyen de préhension.
- La figure 4 illustre, selon une vue en coupe, le dispositif chirurgical illustré sur les figures 1, 2 et 3 dans sa position fonctionnelle expansée, après le retrait du moyen de préhension.
- La figure 4a illustre, selon une vue en coupe, un détail des moyens de blocage anti-retour du dispositif chirurgical illustré sur la figure 3.
- La figure 5 illustre, selon une vue éclatée en perspective, les composants formant le dispositif chirurgical illustré sur les figures 1 à 4.
- La figure 6 illustre, selon une vue de côté en coupe partielle, le kit chirurgical de traitement de la pathologie du pied plat conforme à l'invention.
- La figure 7 illustre, selon une vue en coupe, une variante de réalisation d'un dispositif chirurgical conforme à l'invention, dans une configuration précédant l'expansion diamétrale dudit dispositif.
- La figure 8 illustre, selon une vue en coupe, le dispositif chirurgical illustré sur la figure 7 dans sa configuration fonctionnelle expansée.
- La figure 8a illustre, selon une vue en coupe, un détail des moyens de blocage anti-retour du dispositif chirurgical illustré sur la figure 8.

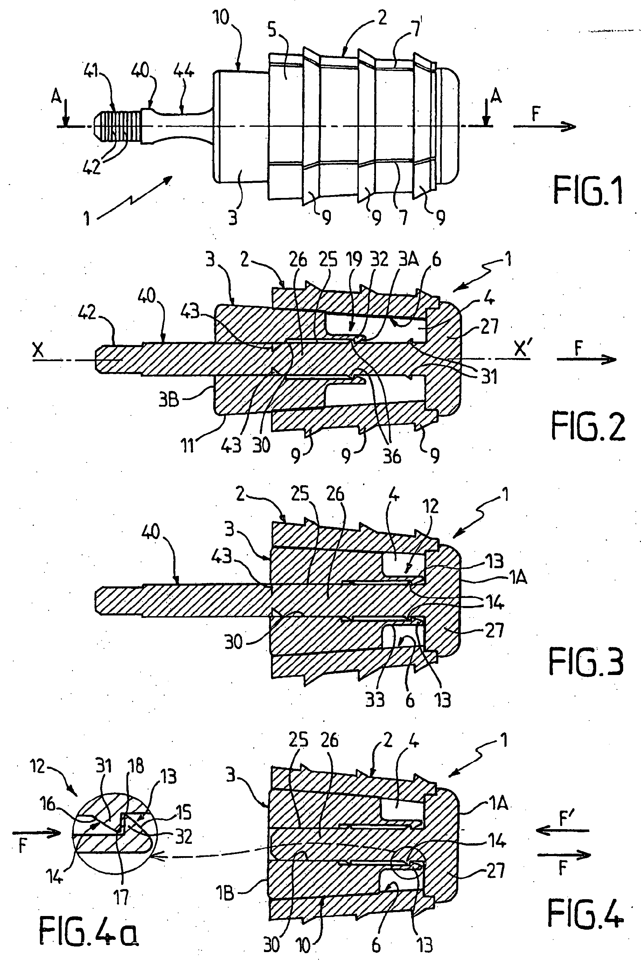

La figure 1 illustre un dispositif chirurgical 1 de traitement de la pathologie du pied plat, avant son montage dans la cavité du sinus tarsien.

Tel que cela est illustré sur la figure 1, le dispositif chirurgical 1 comprend au moins deux composants : une enveloppe externe 2 expansible et un organe d'expansion 3. Avantageusement, l'organe d'expansion 3 a la forme générale d'un cône tronqué s'étendant longitudinalement selon un axe longitudinal X-X' entre une extrémité proximale 3A et une extrémité distale 3B.

L'organe d'expansion 3 est apte à se déplacer au sein de l'enveloppe externe 2 suivant une direction de compression F de préférence parallèle à l'axe longitudinal X-X', l'enveloppe externe 2 et l'organe d'expansion 3 étant conformés pour que le déplacement de l'organe d'expansion 3 suivant la direction de compression F entraîne l'expansion radiale de l'enveloppe externe 2.

L'enveloppe externe 2 se présente avantageusement sous la forme d'une couronne de forme conique dont l'axe principal est confondu avec l'axe longitudinal X-X'. L'enveloppe externe 2 est de préférence formée par un matériau déformable biocompatible, par exemple du métal ou du polyéthylène.

Avantageusement, l'enveloppe externe 2 délimite un logement interne 4 dans lequel vient s'engager l'organe d'expansion 3. L'enveloppe externe 2 comporte avantageusement une surface externe 5 destinée à venir en appui, lors de l'expansion du dispositif chirurgical 1, contre la paroi de la cavité du sinus tarsien. L'enveloppe externe 2. comprend en outre une surface interne 6 délimitant le logement interne 4.

Afin de permettre l'expansion diamétrale de l'enveloppe externe 2, cette dernière possède des entailles 7 disposées sur la surface externe 5, et des entailles 8 disposées sur la surface interne 6. De façon préférentielle, l'enveloppe externe 2 possède quatre entailles 7 réparties sur sa surface externe 5 et quatre entailles 8 réparties sur sa surface interne 6, lesdites entailles 7, 8 étant préférentiellement décalées angulairement les unes par rapport aux autres de manière à permettre l'expansion diamétrale de l'enveloppe externe 2. En particulier, les entailles 7 ménagées sur la surface externe 5 sont préférentiellement décalées de 45° par rapport aux entailles 8 ménagées sur la surface interne 6. Les entailles 7, 8 sont de préférence ménagées longitudinalement, le long de l'enveloppe externe 2, et ont une profondeur préférentiellement supérieure à la moitié de l'épaisseur de l'enveloppe externe 2. Ainsi, lors de l'enfoncement du cône d'expansion 3 au sein du logement 4, les bords des entailles 7, 8 s'écartent, permettant ainsi l'expansion diamétrale de l'enveloppe externe 2. De façon préférentielle, les entailles 7, 8 sont en outre réparties uniformément sur le périmètre de l'enveloppe externe 2 de manière à permettre l'expansion diamétrale homogène du dispositif chirurgical 1.

De façon préférentielle, et tel que cela est illustré sur les figures 1, 2 et 5, l'enveloppe externe 2 comporte des ailettes 9 ménagées sur sa surface externe 5, destinées à s'opposer à l'expulsion du dispositif chirurgical 1 après sa mise en place et son expansion au sein de la cavité du sinus tarsien. Les ailettes 9 viennent ainsi, lors de l'expansion du dispositif chirurgical 1, en appui positif contre la paroi de la cavité, empêchant ainsi l'expulsion du dispositif chirurgical 1.

Avantageusement, l'organe d'expansion 3 comporte un cône d'expansion 10 avec une face externe 11 conformée de manière à venir en appui surfacique contre l'enveloppe externe 2, et préférentiellement contre la surface interne 6 de l'enveloppe externe 2, de manière à provoquer la dilatation progressive de cette dernière sous l'effet du déplacement du cône d'expansion 10 selon la direction de compression F.

De façon préférentielle, l'organe d'expansion 3, et notamment le cône d'expansion 10, est formé par un matériau métallique biocompatible, par exemple du titane ou de l'acier inoxydable.

Le dispositif chirurgical 1 s'étend entre une extrémité proximale 1A qui, dans la position fonctionnelle illustrée sur la figure 4, se trouve au fond de la cavité du sinus tarsien, et une extrémité distale 1B tournée vers l'extérieur de la cavité, à l'opposé de l'extrémité proximale 1A. Avantageusement, le plus petit diamètre du icône d'expansion 10 se trouve du côte de l'extrémité proximale 1A du dispositif chirurgical 1, son plus grand diamètre se trouvant à l'opposé, du côté de l'extrémité distale 1 B du dispositif chirurgical 1.

Avantageusement, la face externe 11, conique, du cône d'expansion 10 vient en appui contre la surface interne 6 du logement interne 4, également de forme conique.

Afin, de permettre l'expansion radiale de l'enveloppe externe 2, le diamètre extérieur du cône d'expansion 10 est sensiblement supérieur au diamètre intérieur correspondant du logement interne 4 lorsque l'enveloppe externe 2 est au repos, c'est-à-dire dans sa configuration non expansée.

Selon une caractéristique essentielle de l'invention, le dispositif chirurgical 1 comporte des moyens de blocage anti-retour 12 conformés pour autoriser le déplacement de l'organe d'expansion 3, selon la direction de compression F, vers une position fonctionnelle au sein de l'enveloppe externe 2 et s'opposer au déplacement en sens inverse de l'organe d'expansion 3 une fois ladite position fonctionnelle atteinte, de manière à empêcher l'expulsion de l'organe d'expansion 3 hors de l'enveloppe externe 2.

En particulier, les moyens de blocage anti-retour 12 ont pour fonction, une fois l'organe d'expansion 3 et l'enveloppe externe 2 assemblés dans la position fonctionnelle, d'empêcher la séparation progressive et incontrôlée des composants (ou pièces) formant le dispositif chirurgical 1, en bloquant l'organe d'expansion 3 dans sa position fonctionnelle, conférant ainsi à ce dernier un caractère indémontable.

Avantageusement, les moyens de blocage anti-retour 12 comportent un premier et un deuxième moyens d'engagement mutuel 13, 14, de préférence respectivement associés, directement ou indirectement, à l'organe d'expansion 3 et à l'enveloppe externe 2, et aptes à coopérer ensemble pour permettre le déplacement unidirectionnel, suivant la direction de compression F, de l'organe d'expansion 3 vers sa position fonctionnelle au sein de l'enveloppe externe 2.

Le terme « *associé »* fait référence au fait que le premier moyen d'engagement mutuel 13 et l'organe d'expansion 3 forment, au cours de l'étape d'expansion du dispositif chirurgical 1, un ensemble unitaire sur le plan cinématique, apte à se déplacer de façon monobloc pour assurer l'expansion de l'enveloppe externe 2. De la même façon, le deuxième moyen d'engagement mutuel 14 est « associé » directement ou indirectement à l'enveloppe externe 2, c'est-à-dire qu'il est soit ménagé directement sur cette dernière, soit lié indirectement, par une pièce intermédiaire, à l'enveloppe externe 2 de manière à être immobile relativement à l'enveloppe externe 2 lors du déplacement de l'organe d'expansion 3, et à former, avec ladite enveloppe externe 2, un ensemble unitaire sur le plan cinématique. Dans la suite de la description, on interprétera l'expression « associé » conformément à la définition énoncée ci-dessus.

Avantageusement, au moins l'un des moyens d'engagement mutuel 13 est conformé et agencé de manière à s'effacer pour franchir l'autre moyen d'engagement mutuel 14 lors du déplacement de l'organe d'expansion 3 suivant la direction de compression F, formant ainsi un moyen d'engagement escamotable. Le moyen d'engagement escamotable peut indifféremment être associé à l'enveloppe externe 2 ou à l'organe d'expansion 3.

De façon préférentielle, le moyen d'engagement escamotable 13 présente un caractère élastique lui permettant de s'effacer vis-à-vis du moyen d'engagement mutuel 14 complémentaire. Ce caractère élastique peut provenir d'un moyen de rappel élastique, du genre ressort, relié mécaniquement au moyen d'engagement escamotable 13 de telle sorte que lorsque le moyen d'engagement mutuel 14 vient en appui contre le moyen d'engagement escamotable 13 correspondant, ce dernier soit repoussé à l'encontre de la force de rappel exercée par le moyen de rappel élastique.

De façon préférentielle, le moyen d'engagement escamotable 13 présente un caractère élastique propre, provenant de sa flexibilité et de sa souplesse intrinsèques.

Tel que cela est illustré sur la figure 4a, les premier et deuxième moyens d'engagement mutuel 13, 14 comportent une première et une deuxième faces d'engagement 15, 16 orientées en oblique par rapport à la direction de compression F, et aptes à glisser l'une contre l'autre lors du déplacement,clé l'organe d'expansion 3 vers sa position fonctionnelle.

De façon préférentielle, les premier et deuxième moyens d'engagement mutuel 13, 14 comportent en outre une première et une deuxième faces d'arrêt 17, 18 aptes à venir en butée l'une contre l'autre de manière à empêcher le retour en arrière, selon une direction d'expulsion F' opposée à la direction de compression F, de l'organe d'expansion 3 une fois la position fonctionnelle atteinte.

Avantageusement, le dispositif chirurgical 1 comporte des moyens de pré-assemblage 19 conformés pour assembler l'organe d'expansion 3 avec l'enveloppe externe 2, de manière directe ou indirecte, par clippage. Cette configuration « *pré-assemblée* » facilite la pose du dispositif chirurgical 1 par le chirurgien, ce dernier n'ayant alors qu'à introduire le dispositif chirurgicale 1, dans sa configuration « pré-assemblée », dans la cavité du sinus tarsien, puis à procéder à l'expansion de l'enveloppe externe 2, par déplacement relatif de l'organe d'expansion 3 et de ladite enveloppe externe 2.

Plusieurs modes de réalisation de l'invention vont maintenant être décrits en se référant aux figures 1 à 5 d'une part et 7 à 8 d'autre part.

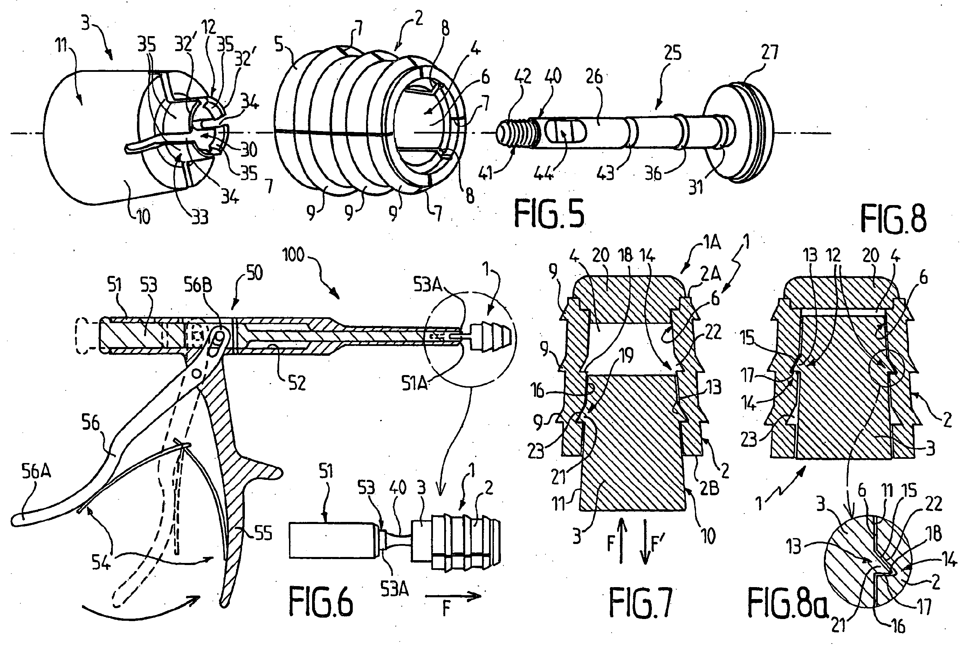

Selon un premier mode de réalisation de l'invention, illustré sur les figures 7 et 8, le dispositif chirurgical 1 est constitué par l'enveloppe externe 2 et l'organe d'expansion 3, formant ainsi un dispositif, chirurgical 1 à deux composants (ou pièces).

Selon cette variante, l'enveloppe externe 2 s'étend entre une extrémité proximale 2A, située vers l'extrémité proximale 1A correspondante du dispositif chirurgical 1 et une extrémité distale 2B, située à l'opposé. Le dispositif chirurgical 1 comporte une tête de blocage 20 en translation, disposée à l'extrémité proximale 2A de l'enveloppe externe 2, de manière à refermer le logement interne 4. La tête de blocage 20 peut être formée par un bouchon, solidarisé ou venu de matière avec l'enveloppe externe 2.

Selon cette variante, l'organe d'expansion 3 est formé par le cône d'expansion 2 qui se présente sous la forme d'un cône tronqué plein. Tel que cela est représenté sur les figures 7, 8 est 8a, le deuxième moyen d'engagement mutuel 14 est situé sur la surface interne 6 du logement interne 4 de manière à coopérer avec le premier moyen d'engagement mutuel 13 situé sur la face externe 11 de l'organe d'expansion 3. La face externe 11 et la surface interne 6 sont, hormis la présence de reliefs formés par des moyens d'engagement mutuel 13, 14, majoritairement lisses.

De façon préférentielle, et tel que cela est illustré sur la figure 8a, le premier moyen d'engagement mutuel 13 est préférentiellement formé par une lèvre circulaire 21, s'étendant sur le pourtour de l'organe d'expansion 3 dans un plan sensiblement perpendiculaire à la direction de compression F. La lèvre circulaire 21 fait avantageusement saillie à partir de la face externe 11 de l'organe de compression 3. Le deuxième moyen d'engagement mutuel 14 est préférentiellement formé par une première rainure circulaire 22, ménagée sur la surface interne 6 de l'enveloppe externe 2, et de forme préférentiellement complémentaire à celle de la lèvre circulaire 21. De façon préférentielle, la lèvre circulaire 21 et la première rainure circulaire 22 possèdent une section transversale triangulaire.

Selon cette variante, les moyens de pré-assemblage 19 sont préférentiellement formés par la lèvre circulaire 21 et au moins une deuxième rainure circulaire 23, ménagée sur la surface interne 6 de l'enveloppe externe 2 et située en amont de la première rainure circulaire 22 en considération de la direction de compression F, ladite lèvre circulaire 21 étant destinée à coopérer avec ladite deuxième rainure circulaire 23 tel que cela est illustré sur la figure 7.

Le fonctionnement du dispositif chirurgical 1 illustré sur les figures 7, 8 et 8a est le suivant.

En se déplaçant selon la direction de compression F, l'organe d'expansion 3 vient, par l'intermédiaire de sa face externe 11 et de la lèvre circulaire 21, en appui positif et compressif sur la surface interne 6 de l'enveloppe externe 2 provoquant ainsi l'expansion diamétrale de cette dernière. Lorsque la configuration de pré-assemblage, illustrée sur la figure 7, est atteinte, la lèvre circulaire 21 vient se loger dans la deuxième rainure circulaire 23 qui empêche le retour en arrière de l'organe d'expansion 3 suivant la direction d'expulsion F'. Dans cette configuration de pré-assemblage, l'organe d'expansion 3 peut néanmoins être déplacé selon la direction de compression F jusqu'à atteindre sa position fonctionnelle illustrée sur la figure 8, dans laquelle il est verrouillé en position d'une part à l'aide des moyens de blocage anti-retour 12, empêchant son déplacement selon la direction d'expulsion F', et d'autre part par la tête de blocage en translation 20, limitant son déplacement en translation suivant la direction de compression F. Dans sa position fonctionnelle, la lèvre circulaire 21 vient se loger dans la première rainure circulaire 22 prévue à cet effet, de telle sorte que les première et deuxième faces d'arrêt 17, 18 viennent en appui l'une contre l'autre.

Selon un deuxième mode de réalisation préférentielle de l'invention illustré sur les figures 1 à 5, le dispositif chirurgical 1 comporte un organe de guidage 25 conformé pour coopérer avec l'organe d'expansion 3 de manière: à guider ce dernier en translation au sein de l'enveloppe externe 2. Plus précisément, l'organe de guidage 25 comporte une tige centrale 26 s'étendant sensiblement au centre de l'enveloppe externe 2, le long de l'axe longitudinal X-X', et l'organe d'expansion 3 comporte un passage central 30 lui permettant de coulisser le long de ladite tige centrale 26. Le dispositif chirurgical 1 est alors constitué par 3 composants, à savoir l'organe d'expansion 3, l'enveloppe externe 2 et l'organe de guidage 25.

Selon une caractéristique particulièrement avantageuse de l'invention, l'organe de guidage 25 comprend en outre une tête de blocage 27 en translation, apte à limiter le déplacement de l'organe d'expansion 3 selon la direction de compression F, en formant une butée à l'encontre de ce dernier. La tête de blocage 27 et les moyens de blocage anti-retour 12 coopèrent alors de manière à assurer le verrouillage en position fonctionnelle de l'organe d'expansion 3.

De façon préférentielle, l'organe de guidage 25 est formé par un matériau métallique biocompatible, tel que le titane ou l'acier inoxydable.

Selon ce deuxième mode de réalisation de l'invention, le deuxième moyen d'engagement mutuel 14 est préférentiellement situé sur le pourtour de la tige centrale 26 de manière à coopérer avec le premier moyen d'engagement 13, situé sur la surface du passage central 30. Le deuxième moyen d'engagement mutuel 14 est alors associé à l'enveloppe externe 2 de manière indirecte, par l'intermédiaire de la pièce intermédiaire formée par l'organe de guidage 25. Le premier moyen d'engagement 13 fait avantageusement saillie dans le passage central 30, à partir de la surface dudit passage central 30.

De façon encore plus préférentielle, et tel que cela est illustré sur la figure 4a, les moyens de blocage anti-retour 12 et notamment les premier et deuxième moyens d'engagement mutuel 13, 14 sont formés par au moins une première lèvre 31, associée à l'enveloppe externe 2 et plus préférentiellement située sur le pourtour de la tige centrale 26 de manière à former une lèvre circulaire, et au moins une contre-lèvre 32 associée à l'organe d'expansion 3, et plus préférentiellement disposée de manière à faire saillie dans le passage central 30.

Avantageusement, et tel que cela est illustré sur les figures 3 et 5, l'organe d'expansion 3 se prolonge, vers l'extrémité proximale 1A du dispositif chirurgical 1, par un col 33 effilé, entourant le passage central 30, la contre-lèvre 32 étant disposée autour du col 33 de manière à faire saillie vers l'intérieur du passage central 30.

De façon particulièrement avantageuse, et tel que cela est illustré sur la figure 5, des fentes 34 sont ménagées le long du col 33 de manière à délimiter plusieurs languettes flexibles 35 aptes à s'effacer à la manière de cliquets vis-à-vis de la première lèvre 31 lors du déplacement de l'organe d'expansion 3 selon la direction de compression F. La contre-lèvre 32 se décompose ainsi en une pluralité de contre-lèvres 32', disposées sur les languettes flexibles 35 et forme avantageusement, en association avec la première lèvre 31, un encliquetage.

Le pourtour de la tige centrale 26 et la surface du passage central 30 sont de préférence majoritairement lisses, excepté la présence des moyens d'engagement mutuel 13, 14 qui forment des reliefs.

Selon ce mode de réalisation de l'invention, les moyens de pré-assemblage 19 sont de préférence formés par la contre-lèvre 32 et au moins une deuxième lèvre 36, associée à l'enveloppe externe 2, et plus précisément ménagée sur le pourtour de la tige centrale 26 et située en amont de la première lèvre 31 en considération de la direction de compression F, ladite contre-lèvre 32 étant destinée à coopérer avec ladite deuxième lèvre 36 dans la configuration de pré-assemblage illustrée sur la figure 2.

Selon une caractéristique particulièrement avantageuse de l'invention, illustrée sur les figures 1 à 3 et 5, le dispositif chirurgical 1 comporte un moyen de préhension 40, destiné à être raccordé mécaniquement à un instrument de pose 50 (figure 6) conçu pour assurer le clippage (ou l'encliquetage) des composants formant le dispositif chirurgical 1 en exerçant un effort de traction sur le moyen de préhension 40, de préférence suivant une direction opposée à la direction de compression F. De façon préférentielle, le moyen de préhension 40 comporte un embout 41 pourvu d'un filetage externe 42 permettant son raccordement par vissage avec l'instrument de pose 50 (figure 5).

Le moyen de préhension 40 est préférentiellement formé par un prolongement de la tige centrale 26 situé à l'opposé de la tête de blocage 27. Le moyen de préhension 40 est en outre avantageusement amovible et de préférence sécable. A cet effet, la tige centrale 26 et le moyen de préhension 40 sont avantageusement formés par une même pièce pourvue d'un rétrécissement local 43 définissant une zone de rupture au niveau de laquelle s'effectue la séparation du moyen de préhension 40 et de la tige centrale 26 lorsqu'un effort de traction dépassant une valeur seuil est exercé sur le moyen de préhension 40.

De façon particulièrement avantageuse, la zone de rupture, et notamment le rétrécissement local 43, est située pendant toute l'étape d'expansion du dispositif chirurgical 1 dans le passage central 30 de l'organe d'expansion 3, qui empêche une rupture prématurée du moyen de préhension 40, notamment en s'opposant à la flexion et au débattement angulaire du moyen de préhension 40 par rapport à l'axe longitudinal X-X' et à la tige centrale 26.

La rupture effective ne peut avoir lieu que lorsque l'organe d'expansion 3 a atteint sa position fonctionnelle au sein de l'enveloppe externe 2, la zone de rupture venant dans ce cas affleurer l'extrémité distale 3B de l'organe d'expansion 3 de manière à permettre le sectionnement du moyen de préhension 40, notamment en tirant sur ce dernier et en l'inclinant par rapport à l'axe longitudinal X-X' au niveau du rétrécissement local 43.

Le dispositif chirurgical 1 conforme à l'invention est avantageusement dimensionné pour que lorsque l'organe d'expansion 3 se trouve dans sa position fonctionnelle, la zone de rupture (ou le rétrécissement local 43) se trouve sensiblement au niveau de l'extrémité distale 3B de l'organe d'expansion 3, située à l'opposé de la tête de blocage 27.

Avantageusement, le moyen de préhension 40 comporte un méplat 44 destiné à faciliter sa préhension par le chirurgien pour assurer le dévissage de l'embout 41 vis à vis de l'instrument de pose 50.

La présente invention concerne également un kit chirurgical 100 de traitement de la pathologie du pied plat comportant :
- un dispositif chirurgical 1 tel que décrit précédemment, comprenant un moyen de préhension 40,
- un instrument de pose 50, apte à être raccordé mécaniquement au moyen de préhension 40 et à exercer un effort de traction sur ce dernier, tout en repoussant l'organe d'expansion 3 selon la direction de compression F, de telle sorte que ce dernier pénètre progressivement à intérieur de l'enveloppe externe 2 en provoquant son expansion, et notamment son expansion diamétrale.

Tel que cela est illustré sur la figure 6, l'instrument de pose 50 comporte un corps principal 51, de forme cylindrique, pourvu d'une cavité 52 dans laquelle est montée une tige 53. La tige 53 est monté mobile en translation au sein de la cavité 52 et comporte une extrémité libre 53A, sur laquelle est monté le dispositif chirurgical 1. Ce dernier est par exemple vissé sur l'extrémité libre 53A de la tige 53. Le déplacement de la tige 53 suivant la direction de compression F est commandé par des poignées 54, et notamment par une poignée fixe 55, solidarisée à ou venue de matière avec le corps principal 51 et une poignée mobile 56, montée avec une possibilité de rotation par rapport au corps principal 51. La poignée mobile 56 s'étend entre une première extrémité libre 56A et une deuxième extrémité 56B reliée mécaniquement à la tige 53, de telle sorte qu'un mouvement de rotation de la poignée mobile 56 entraîne un mouvement de translation correspondant de la tige 53, qui coulisse dans le corps principal 51. L'instrument de pose 50 forme ainsi avantageusement une « *pince à rivet* ».

La présente invention concerne enfin une méthode chirurgical de traitement de la pathologie du pied plat comprenant tout d'abord une étape d'implantation, au cours de laquelle on introduit un dispositif chirurgical 1, comprenant une enveloppe externe 2 expansible, dans le sinus du tarse du pied. La méthode chirurgicale conforme à l'invention comprend ensuite une étape d'expansion, au cours de laquelle on introduit et on déplace un organe d'expansion 3 dans l'enveloppe externe 2 de manière à provoquer son expansion.

La méthode chirurgical conforme à l'invention comprend en outre, selon une caractéristique essentielle, une étape de blocage au cours de laquelle on empêche, à l'aide de moyens de blocage anti-retour 12, l'expulsion de l'organe d'expansion 3 hors de l'enveloppe externe 2 une fois sa position fonctionnelle au sein de l'enveloppe externe 2 atteinte.

De façon particulièrement avantageuse, la méthode conforme à l'invention comporte, avant l'étape d'expansion, une première étape de pré-assemblage, au cours de laquelle on assemble, directement, ou indirectement, par exemple à l'aide d'une pièce intermédiaire, l'organe d'expansion 3 avec l'enveloppe externe 2 de manière à former un ensemble unitaire indémontable. L'étape de pré-assemblage s'effectue avantageusement par clippage, à l'aide de moyens de pré-assemblage. Cette étape est de préférence réalisée sur le site de production du dispositif chirurgical 1, ce dernier étant de préférence vendu dans sa configuration pré-assemblée.

Avantageusement, la méthode chirurgicale conforme à l'invention comprend également, préalablement à l'étape d'expansion, une étape de raccordement, au cours de laquelle on vient raccorder mécaniquement le dispositif chirurgical 1 à un instrument de pose 50 par l'intermédiaire d'un moyen de préhension 40, intégré au dispositif chirurgical 1.

L'étape d'expansion s'effectue alors par traction sur le moyen de préhension 40, à l'aide de l'instrument de pose 50, de telle sorte que l'effort de traction exercé sur le moyen de préhension 40 entraîne le déplacement de l'organe d'expansion 3 dans l'enveloppe externe 2 et l'expansion diamétrale de cette dernière. L'étape d'expansion se poursuit jusqu'au blocage en position de l'organe d'expansion, à l'aide des moyens de blocage anti-retour 12. Selon une caractéristique particulièrement avantageuse de l'invention, l'étape de blocage s'effectue par clippage ou encliquetage de l'organe d'expansion 3 avec l'enveloppe externe 2, et ce de manière directe, ou indirecte c'est-à-dire par l'intermédiaire d'une pièce intermédiaire telle qu'un organe de guidage 25. Dans ce dernier cas, l'organe de guidage 25 comporte avantageusement une tête de blocage 27 qui, en association avec les moyens de blocage anti-retour 12, participe au verrouillage du dispositif chirurgical 1 en immobilisant l'organe d'expansion 3 entre la tête de blocage 27 d'une part et les moyens de blocage anti-retour 12 d'autre part.

La méthode chirurgicale conforme à l'invention comprend en outre, de façon particulièrement avantageuse, une étape de séparation, au cours de laquelle on sépare, par exemple par sectionnement, le moyen de préhension 40.du dispositif chirurgical 1, une fois l'étape d'expansion et l'étape de blocage réalisées.

Les modes de fonctionnement et d'utilisation du dispositif chirurgical 1 et du kit chirurgical 100 conformes à l'invention vont maintenant être décrits en se référant aux figures 1 à 6.

L'insertion du dispositif chirurgical 1 s'effectue de la façon suivante. On pratique une incision unique au niveau du canal tarsien et on introduit l'ensemble des composants formant le dispositif chirurgical selon l'invention dans une configuration pré-assemblée, telle qu'illustrée sur la figure 2.

Le dispositif chirurgical 1 est raccordé à l'instrument de pose 50 par l'intermédiaire du moyen de préhension 40, qui fait saillie à l'extérieur du dispositif chirurgical 1. Plus précisément, l'embout 41 du moyen de préhension 40 est préférentiellement vissé à l'extrémité libre 53A de la tige 53 de l'instrument de pose 50.

Une fois le dispositif chirurgical 1 implanté, on procède à son expansion et à son blocage dans la cavité du sinus tarsien en pressant les poignées 55, 56 l'une contre l'autre de manière à exercer un effort de traction sur le moyen de préhension 40 (et donc sur l'enveloppe externe 2) par l'intermédiaire de la tige 53, tout en poussant l'organe d'expansion 3 suivant la direction de compression F par l'intermédiaire de l'extrémité 51A du corps principal 51, qui vient en appui contre l'organe d'expansion 3.

L'organe d'expansion 3, et notamment le cône d'expansion 10, pénètre alors progressivement dans le logement interne 4 de l'enveloppe externe 2 et exerce une compression centrifuge sur cette dernière en raison de la différence de diamètre entre le cône d'expansion 10 et le logement interne 4. Plus précisément, au fur et à mesure de l'expansion de l'enveloppe externe 2, les entailles 7, 8 s'ouvrent progressivement et les ailettes 9 viennent en appui contre la cavité du sinus tarsien, bloquant ainsi le dispositif chirurgical 1 au sein de ladite cavité.

La position fonctionnelle de l'organe d'expansion 3 est atteinte lorsque ce dernier vient en butée contre la tête de blocage 27, notamment par l'intermédiaire du col 33 (figure 3) et/ou lorsque les premier et deuxième moyens d'engagement mutuel 13, 14 viennent s'encliqueter l'un avec l'autre. L'organe d'expansion 3 est alors verrouillé en position, conférant ainsi un caractère indémontable au dispositif chirurgical 1.

Le chirurgien peut alors poursuivre l'effort de traction exercé sur le moyen de préhension 40, en pressant les poignées 55, 56 l'une contre l'autre, tout en inclinant l'instrument de pose 50 de manière à séparer, par rupture en flexion et en traction au niveau du rétrécissement local 43, le moyen de préhension 40 de la tige centrale 26 et plus généralement du dispositif chirurgical 1 qui se trouve alors bloqué en position dans la cavité du sinus tarsien.

La présente invention permet une mise en place et un blocage faciles du dispositif chirurgical 1 dans le sinus du tarse, à l'aide d'un nombre limité d'opérations, tout en offrant une bonne fiabilité et notamment un risque d'expulsion limité du dispositif chirurgical 1 ou de l'un de ses composants.

## Revendications

1. Dispositif chirurgical (1) de traitement de la pathologie du pied plat comprenant au moins deux composants :
- une enveloppe externe (2) expansible,
- un organe d'expansion (3), apte à se déplacer au sein de ladite enveloppe externe (2) suivant une direction de compression (F), ladite enveloppe externe (2) et ledit organe d'expansion (3) étant conformés pour que le déplacement de l'organe d'expansion (3) suivant la direction de compression (F) entraîne l'expansion radiale de ladite enveloppe externe (2),
**caractérisé en ce qu'**il comporte des moyens de blocage anti-retour (12), conformés pour autoriser le déplacement de l'organe d'expansion(3), selon la direction de compression (F), vers une position fonctionnelle au sein de l'enveloppe externe (2) et s'opposer au déplacement en sens inverse de l'organe d'expansion (3) une fois ladite position fonctionnelle atteinte, de manière à empêcher l'expulsion de l'organe d'expansion (3).

2. Dispositif selon la revendication 1 **caractérisé en ce que** les moyens de blocage anti-retour (12) comportent un premier et un deuxième moyens d'engagement mutuel (13, 14), respectivement associés à l'organe d'expansion (3) et à l'enveloppe externe (2), et aptes à coopérer ensemble pour permettre le déplacement unidirectionnel de l'organe d'expansion (3) vers sa position fonctionnelle.

3. Dispositif selon la revendication 2 **caractérisé en ce qu'**au moins l'un desdits moyens d'engagement mutuel (13, 14) est conformé et agencé de manière à s'effacer pour franchir l'autre moyen d'engagement mutuel lors du déplacement de l'organe d'expansion (3) suivant la direction de compression (F), formant ainsi un moyen d'engagement escamotable.

4. Dispositif selon la revendication 3 **caractérisé en ce que** le moyen d'engagement escamotable (13) présente un caractère élastique.

5. Dispositif selon l'une des revendications 2 à 4 **caractérisé en ce que** les premier et deuxième moyens d'engagement mutuel (13, 14) comportent une première et une deuxième faces d'engagement (15, 16), orientées en oblique par rapport à ,là direction de compression (F), et aptes à glisser l'une contre l'autre lors du déplacement de l'organe d'expansion (3) vers sa position fonctionnelle.

6. Dispositif selon l'une des revendications 2 à 5 **caractérisé en ce que** les premier et deuxième moyens d'engagement mutuel (13, 14) comportent une première et une deuxième faces d'arrêt (17, 18), aptes à venir en butée l'une contre l'autre de manière à empêcher le retour en arrière de l'organe d'expansion (3) une fois la position fonctionnelle atteinte.

7. Dispositif selon l'une des revendications précédentes **caractérisé en ce que** les moyens de blocage anti-retour (12) sont formés par au moins une première lèvre (31), associée à l'enveloppe externe (2) et au moins une contre-lèvre (32) associée à l'organe d'expansion (3).

8. Dispositif selon l'une des revendications précédentes **caractérisé en ce qu'**il comporte des moyens de pré-assemblage (19), conformés pour assembler l'organe d'expansion (3) avec l'enveloppe externe (2) par clippage.

9. Dispositif selon les revendications 7 et 8 **caractérisé en ce que** les moyens de pré-assemblage (19) sont formés par la contre-lèvre (32) et au moins une deuxième lèvre (36), associée à l'enveloppe externe (2) et située en amont de la première lèvre (31) en considération de la direction de compression (F), ladite contre-lèvre (32) étant destinée à coopérer avec ladite deuxième lèvre (36).

10. Dispositif selon l'une des revendications 2 à 9 **caractérisé en ce que** l'organe d'expansion (3) comporte un cône d'expansion (10), la face externe (11) du cône d'expansion (10) venant en appui surfacique contre l'enveloppe externe (2) de manière à provoquer la dilatation progressive de cette dernière sous l'effet du déplacement du cône d'expansion (10) selon la direction de compression (F).

11. Dispositif selon l'une des revendications 2 à 10 **caractérisé en ce qu'**il est constitué par l'enveloppe externe (2) et l'organe d'expansion (3), formant ainsi un dispositif à deux composants.

12. Dispositif selon les revendications 2 et 11 **caractérisé en ce que** l'enveloppe externe (2) délimite un logement interne (4) avec une surface interne (6), et **en ce que** le deuxième moyen d'engagement (14) est situé sur ladite surface interne (6) de manière à coopérer avec le premier moyen d'engagement (13), situé sur la face externe (11) de l'organe d'expansion (3).

13. Dispositif selon l'une des revendications 2 à 10 **caractérisé en ce qu'**il comporte un organe de guidage (25), conformé pour coopérer avec l'organe d'expansion (3) de manière à guider ce dernier en translation au sein de l'enveloppe externe (2).

14. Dispositif selon la revendication 13 **caractérisé en ce que** l'organe de guidage (25) comporte une tige centrale (26), s'étendant sensiblement au centre de l'enveloppe externe (2), et **en ce que** l'organe d'expansion (3) comporte un passage central (30) lui permettant de coulisser le long de la tige centrale (26).

15. Dispositif selon la revendication 13 ou 14 **caractérisé en ce que** l'organe de guidage (25) comporte une tête de blocage (27) en translation, apte à limiter le déplacement de l'organe d'expansion (3) selon la direction de compression (F), la tête de blocage (27) et les moyens de blocage anti-retour (12) assurant le verrouillage en position fonctionnelle de l'organe d'expansion (3).

16. Dispositif selon la revendication 14 **caractérisé en ce que** le deuxième moyen d'engagement (14) est situé sur le pourtour de la tige centrale (26), de marnière à coopérer avec lie premier moyen d'engagement (13) faisant saillie dans le passage central (30).

17. Dispositif selon les revendications 7 et 16 **caractérisé en ce que** l'organe d'expansion (3) se prolonge par un col (33) effilé, entourant le passage central (30), la contre-lèvre (32) étant disposée au niveau dudit col (33) de manière à faire saillie vers l'intérieur du passage central (30).

18. Dispositif selon la revendication 17 **caractérisé en ce que** des fentes (34) sont ménagées le long du col (33) de manière à délimiter plusieurs languettes flexibles (35) aptes à s'effacer vis-à-vis de la première lèvre (31) lors du déplacement de l'organe d'expansion (3) selon la direction de compression (F).

19. Dispositif selon l'une des revendications précédentes **caractérisé en ce qu'**il comporte un moyen de préhension (40), destiné à être raccordé mécaniquement à un instrument de pose (50) conçu pour assurer le clippage des composants formant le dispositif chirurgical en exerçant un effort de traction sur le moyen de préhension (40).

20. Dispositif selon les revendications 14, 15 et 19 **caractérisé en ce que** le moyen de préhension (40) est formé par un prolongement de la tige centrale (26), situé à l'opposé de la tête de blocage (27).

21. Dispositif selon l'une des revendications 19 ou 20 **caractérisé en ce que** le moyen de préhension (40) est amovible, de préférence sécable.

22. Dispositif selon la revendication 21 **caractérisé en ce que** la tige centrale (26) et le moyen de préhension (40) sont formés par une même pièce pourvue d'un rétrécissement local (43) définissant .une zone de rupture au niveau de laquelle s'effectue la séparation du moyen de préhension (40) de la tige centrale (26) lorsqu'un effort de traction dépassant une valeur seuil est exercé sur le moyen de préhension (40).

23. Dispositif selon l'une des revendications précédentes **caractérisé en ce que** l'enveloppe externe (2) possède des entailles (7, 8) sur sa surface externe (5) et sur sa surface interne (6), décalées angulairement de manière à permettre l'expansion diamétrale de ladite enveloppe externe (2).

24. Dispositif selon l'une des revendications précédentes **caractérisé en ce que** l'enveloppe externe (2) est formée par un matériau déformable biocompatible, par exemple du métal ou du polyéthylène.

25. Kit chirurgical de traitement de la pathologie du pied plat comportant :
- un dispositif chirurgical (1) selon l'une des revendications 1 à 24, comprenant un moyen de préhension (40),
- un instrument de pose (50), apte à être raccordé mécaniquement au moyen de préhension (40) et à exercer un effort de traction sur ce dernier, tout en repoussant l'organe d'expansion (3) selon la direction de compression (F) de telle sorte que ce dernier pénètre progressivement à l'intérieur de l'enveloppe externe (2) en provoquant son expansion.

## Patentansprüche

1. Chirurgische Vorrichtung (1) zum Behandeln von Plattfüßen, die mindestens zwei Komponenten aufweist:
- eine äußere ausdehnbare Hülle (2),
- ein Ausdehnungsorgan (3), das geeignet ist, sich in der äußeren Hülle (2) gemäß einer Kompressionsrichtung (F) zu verschieben, wobei die äußere Hülle (2) und das Ausdehnungsorgan (3) so ausgebildet sind, dass die Verschiebung des Ausdehnungsorgans (3) gemäß der Kompressionsrichtung (F) eine radiale Ausdehnung der äußeren Hülle (2) nach sich zieht,
**dadurch gekennzeichnet, dass** sie Rücklaufsperrenmittel (23) aufweist, die so ausgebildet sind, dass sie die Verschiebung des Ausdehnungsorgans (3) gemäß der Kompressionsrichtung (F) hin zu einer funktionalen Position in der äußeren Hülle (2) hin gestatten und sich der Verschiebung des Ausdehnungsorgans (3) in entgegengesetzter Richtung widersetzen, sobald die funktionale Position erreicht ist, sodass das Ausstoßen des Ausdehnungsorgans (3) verhindert wird.

2. Vorrichtung nach Anspruch 1, **dadurch gekennzeichnet, dass** die Rücklaufsperrenmittel (12) ein erstes und ein zweites Mittel für ein gegenseitiges Eingreifen (13, 14) aufweisen, die jeweils mit dem Ausdehnungsorgan (3) und mit der äußeren Hülle (2) verbunden sind und geeignet sind, miteinander zusammenzuwirken, um die unidirektionale Verschiebung des Ausdehnungsorgans (3) zu seiner funktionalen Position hin zu gestatten.

3. Vorrichtung nach Anspruch 2, **dadurch gekennzeichnet, dass** mindestens eines der Mittel für ein gegenseitiges Eingreifen (13, 14) so ausgebildet und angeordnet ist, dass es zurückweicht, um das andere Mittel für ein gegenseitiges Eingreifen während der Verschiebung des Ausdehnungsorgans (3) gemäß der Kompressionsrichtung (F) zu überwinden, und somit ein einziehbares Eingreifmittel bildet.

4. Vorrichtung nach Anspruch 3, **dadurch gekennzeichnet, dass** das einziehbare Eingreifmittel (13) ein elastisches Merkmal aufweist.

5. Vorrichtung nach einem der Ansprüche 2 bis 4, **dadurch gekennzeichnet, dass** das erste und das zweite Mittel für ein gegenseitiges Eingreifen (13, 14) eine erste und eine zweite Eingreiffläche (15, 16) aufweisen, die in Bezug auf die Kompressionsrichtung (F) schrägliegend ausgerichtet sind und geeignet sind, während der Verschiebung des Ausdehnungsorgans (3) zu seiner funktionalen Position hin gegeneinander zu gleiten.

6. Vorrichtung nach einem der Ansprüche 2 bis 5, **dadurch gekennzeichnet, dass** das erste und das zweite Mittel für ein gegenseitiges Eingreifen (13, 14) eine erste und eine zweite Stoppfläche (17, 18) aufweisen, die geeignet sind, gegeneinander so zum Anschlag zu kommen, dass der Rücklauf des Ausdehnungsorgans (3) verhindert wird, sobald es die funktionale Position erreicht hat.

7. Vorrichtung nach einem der vorhergehenden Ansprüche, **dadurch gekennzeichnet, dass** die Rücklaufsperrenmittel (12) durch mindestens eine erste Lippe (31) gebildet werden, die mit der äußeren Hülle (2) verbunden ist, und durch mindestens eine Gegenlippe (32), die mit dem Ausdehnungsorgan (3) verbunden ist.

8. Vorrichtung nach einem der vorhergehenden Ansprüche, **dadurch gekennzeichnet, dass** sie Mittel zum Vorab-Zusammensetzen (19) aufweist, die so ausgebildet sind, dass sie das Ausdehnungsorgan (3) mit der äußeren Hülle (2) mittels Rastverbindung zusammensetzen.

9. Vorrichtung nach den Ansprüchen 7 und 8, **dadurch gekennzeichnet, dass** die Mittel zum Vorab-Zusammensetzen (19) durch die Gegenlippe (32) und mindestens eine zweite Lippe (36) gebildet werden, die mit der äußeren Hülle (2) verbunden ist und unter Berücksichtigung der Kompressionsrichtung (F) vor der ersten Lippe (31) angeordnet ist, wobei die Gegenlippe (32) dazu bestimmt ist, mit der zweiten Lippe (36) zusammenzuwirken.

10. Vorrichtung nach einem der Ansprüche 2 bis 9, **dadurch gekennzeichnet, dass** das Ausdehnungsorgan (3) einen Ausdehnungskegel (10) aufweist, wobei die Außenfläche (11) des Ausdehnungskegels (10) mit der Oberfläche auf der äußeren Hülle (2) so zum Aufliegen kommt, dass die zunehmende Ausdehnung dieser Letzteren unter der Einwirkung der Verschiebung des Ausdehnungskegels (10) gemäß der Kompressionsrichtung (F) herbeigeführt wird.

11. Vorrichtung nach einem der Ansprüche 2 bis 10, **dadurch gekennzeichnet, dass** sie durch die äußere Hülle (2) und das Ausdehnungsorgan (3) gebildet wird, die dadurch eine Vorrichtung mit zwei Komponenten bilden.

12. Vorrichtung nach den Ansprüchen 2 und 11, **dadurch gekennzeichnet, dass** die äußere Hülle (2) eine interne Aufnahme (4) mit einer Innenfläche (6) begrenzt, und dadurch, dass das zweite Eingreifmittel (14) sich so auf der Innenfläche (6) befindet, dass es mit dem ersten Eingreifmittel (13) zusammenwirkt, das sich auf der Außenfläche (11) des Ausdehnungsorgans (3) befindet.

13. Vorrichtung nach einem der Ansprüche 2 bis 10, **dadurch gekennzeichnet, dass** sie ein Führungsorgan (25) aufweist, das so ausgebildet ist, dass es mit dem Ausdehnungsorgan (3) so zusammenwirkt, dass es dieses Letztere parallel verschiebbar in der äußeren Hülle (2) führt.

14. Vorrichtung nach Anspruch 13, **dadurch gekennzeichnet, dass** das Führungsorgan (25) einen zentralen Stab (26) aufweist, der sich im Wesentlichen in der Mitte der äußeren Hülle (2) erstreckt, und dadurch, dass das Ausdehnungsorgan (3) eine zentrale Durchführung (30) aufweist, die gestattet, dass es den zentralen Stab (26) entlang gleitet.

15. Vorrichtung nach Anspruch 13 oder 14, **dadurch gekennzeichnet, dass** das Führungsorgan (25) einen parallel verschiebbaren Sperrenkopf (27) aufweist, der geeignet ist, die Verschiebung des Ausdehnungsorgans (3) gemäß der Kompressionsrichtung (F) zu begrenzen, wobei der Sperrenkopf (27) und die Rücklaufsperrenmittel (12) die Verriegelung des Ausdehnungsorgans (3) in der funktionalen Position sicherstellen.

16. Vorrichtung nach Anspruch 14, **dadurch gekennzeichnet, dass** das zweite Eingreifmittel (14) sich so auf dem Umfang des zentralen Stabs (26) befindet, dass es mit dem ersten Eingreifmittel (13) zusammenwirkt, das in der zentralen Durchführung (30) vorspringt.

17. Vorrichtung nach den Ansprüchen 7 und 16, **dadurch gekennzeichnet, dass** das Ausdehnungsorgan (3) sich durch einen zugespitzten Kragen (33) verlängert, der die zentrale Durchführung (30) umgibt, wobei die Gegenlippe (32) auf der Höhe des Kragens (33) so angeordnet ist, dass sie in das Innere der zentralen Durchführung (30) vorspringt.

18. Vorrichtung nach Anspruch 17, **dadurch gekennzeichnet, dass** Schlitze (34) entlang des Kragens (33) so vorgesehen sind, dass sie mehrere biegsame Laschen (35) begrenzen, die geeignet sind, gegenüber der ersten Lippe (31) während der Verschiebung des Ausdehnungsorgans (3) gemäß der Kompressionsrichtung (F) zurückzuweichen.

19. Vorrichtung nach einem der vorhergehenden Ansprüche, **dadurch gekennzeichnet, dass** sie ein Greifmittel (40) aufweist, das dazu bestimmt ist, mechanisch mit einem Verlegeinstrument (50) verbunden zu werden, das so konzipiert ist, dass es die Rastverbindung der Komponenten sicherstellt, welche die chirurgische Vorrichtung bilden, indem eine Zugwirkung auf das Greifmittel (40) ausgeübt wird.

20. Vorrichtung nach den Ansprüchen 14, 15 und 19, **dadurch gekennzeichnet, dass** das Greifmittel (40) durch eine Verlängerung des zentralen Stabs (26) gebildet wird, die sich am entgegengesetzten Ende des Sperrenkopfs (27) befindet.

21. Vorrichtung nach einem der Ansprüche 19 oder 20, **dadurch gekennzeichnet, dass** das Greifmittel (40) abnehmbar ist, vorzugsweise teilbar.

22. Vorrichtung nach Anspruch 21, **dadurch gekennzeichnet, dass** der zentrale Stab (26) und das Greifmittel (40) aus einem einzigen Stück gebildet werden, das mit einer lokalen Verengung (43) versehen ist, die eine Bruchzone definiert, in der die Trennung des Greifmittels (40) des zentralen Stabs (26) erfolgt, wenn eine Zugkraft, die einen Schwellenwert überschreitet, auf das Greifmittel (40) ausgeübt wird.

23. Vorrichtung nach einem der vorhergehenden Ansprüche, **dadurch gekennzeichnet, dass** die äußere Hülle (2) auf der Außenfläche (5) und auf der Innenfläche (6) Einkerbungen (7, 8) besitzt, die winklig so versetzt sind, dass sie eine diametrale Ausdehnung der äußeren Hülle (2) gestatten.

24. Vorrichtung nach einem der vorhergehenden Ansprüche, **dadurch gekennzeichnet, dass** die äußere Hülle (2) aus einem verformbaren, biologisch verträglichen Material gebildet wird, zum Beispiel aus Metall oder Polyethylen.

25. Chirurgischer Teilesatz zum Behandeln von Plattfüßen, aufweisend:
- eine chirurgische Vorrichtung (1) nach einem der Ansprüche 1 bis 24, die ein Greifmittel (40) aufweist,
- ein Verlegeinstrument (50), das geeignet ist, mechanisch mit dem Greifmittel (40) verbunden zu werden und eine Zugkraft auf das Letztere auszuüben, wobei gleichzeitig das Ausdehnungsorgan (3) gemäß der Kompressionsrichtung (F) so zurückgedrückt wird, dass dieses Letztere zunehmend in das Innere der externen Hülle (2) eindringt, wodurch ihre Ausdehnung herbeigeführt wird.

## Claims

1. A surgical device (1) for treating the pathology of flat feet, said surgical device comprising at least two components:
an expansible outer casing (2); and
an expansion member (3) suitable for moving inside said outer casing (2) in a compression direction, said outer casing (2) and said expansion member (3) being shaped so that moving the expansion member (3) in the compression direction (F) causes said outer casing (2) to expand radially, **characterized in that** it comprises non-return check means (12) shaped to allow the expansion member (3) to move in the compression direction (F) towards an operating position inside the outer casing (2), and to oppose movement of the expansion member (3) in the reverse direction once said operating position is reached, so as to prevent the expansion member (3) from being expelled.

2. A device according to claim 1, wherein the check means (12) comprise first and second mutual engagement means (13, 14) associated respectively with the expansion member (3) and with the outer casing (2), and suitable for co-operating together to enable the expansion member (3) to move one-way only, towards its operating position.

3. A device according to claim 2, wherein at least one of said mutual engagement means (13, 14) is shaped and arranged so as to retract in order to go past the other mutual engagement means while the expansion member (3) is moving in the compression direction (F), thereby forming retractable engagement means.

4. A device according to claim 3, wherein the retractable engagement means (13) are resilient.

5. A device according to anyone of claims 2 to 4, wherein the first and second mutual engagement means (13, 14) have first and second engagement faces (15, 16) extending slantingly relative to the compression direction (F) and suitable for sliding against each other while the expansion member (3) is moving towards its operating position.

6. A device according to anyone of claims 2 to 5, wherein the first and second mutual engagement means (13, 14) have first and second stop faces (17, 18) suitable for coming into abutment against each other so as to prevent the expansion member (3) from moving back the other way once the operating position is reached.

7. A device according to anyone of preceding claims, wherein the check means (12) are formed by at least a first lip (31) associated with the outer casing (2) and by at least one backing lip (32) associated with the expansion member (3).

8. A device according to anyone of preceding claims, the device being provided with preassembly means (19) shaped to assemble the expansion member (3) to the outer casing (2) by clipping.

9. A device according to claims 7 and 8, wherein preassembly means (19)are formed by the backing lip (32) and by at least a second lip (36) associated with the outer casing (2) and situated upstream from the first lip (31) relative to the compression direction (F), said backing lip (32) serving to co-operate with said second lip (36).

10. A device according to anyone of claims 2 to 9, wherein the expansion member (3) comprises an expansion cone (10), the outside face (11) of the expansion cone (10) coming into surface abutment against the outer casing (2) so as to cause said outer casing to expand progressively under the effect of the expansion cone (10) moving in the compression direction (F).

11. A device according to anyone of claims 2 to 10, the device being constituted by the outer casing (2) and by the expansion member (3), thereby forming a device made up of two components.

12. A device according to claims 2 and 11, wherein the outer casing (2) defines an internal recess (4) with an inside surface (6), and wherein the second engagement means (14) are situated on said inside surface (6) so as to co-operate with the first engagement means (13), situated on the outside face (11) of the expansion member (3).

13. A device according to anyone of claims 2 to 10, further comprising a guide member (25) shaped to co-operate with the expansion member (3) so as to guide said expansion member in translation inside the outer casing (2).

14. A device according to claim 13, wherein the guide member (25) comprises a central rod (26) extending substantially at the center of the outer casing (2), and wherein the expansion member (3) is provided with a central passageway (30) enabling it to slide along the central rod (26).

15. A device according to claim 13 or 14, wherein the guide member (25) further comprises a blocking head (27) suitable for limiting movement in translation of the expansion member (3) in the compression direction (F), the blocking head (27) and the check means (12) locking the expansion member (3) in the operating position.

16. A device according to claim 14, wherein the second engagement means (14) are situated on the periphery of the central rod (26), so as to co-operate with the first engagement means (13) that project into the central passageway (30).

17. A device according to claims 7 and 16, wherein the expansion member (3) is extended by a thin neck (33) surrounding the central passageway (30), the backing lip (31) being disposed at said neck (33) so as to project towards the inside of the central passageway (30).

18. A device according to claim 17, wherein slots (34) are provided along the neck (33) so as to define a plurality of flexible tongues (35) suitable for retracting relative to the first lip (31) while the expansion member (3) is moving in the compression direction (F).

19. A device according to anyone of preceding claims, further comprising a graspable member (40) serving to be connected mechanically to a fitting instrument (50) designed to clip together the components making up the surgical device by exerting a traction force on the graspable means (40).

20. A device according to claims 14, 15 and 19, wherein the graspable means (40) are formed by an extension to the central rod (26), that is situated opposite from the blocking head (27).

21. A device according to anyone of claims 19 or 20, wherein the graspable means (40) are removable, preferably by being suitable for being broken off.

22. A device according to claim 21, wherein the central rod (26) and the graspable means (40) are formed in one piece provided with a local narrow portion (43) defining a break zone at which the graspable means (40) are separated from the central rod (26) when traction exceeding a threshold value is exerted on the graspable means (40).

23. A device according to anyone of preceding claims, wherein the outer casing (2) is provided with notches (7, 8) in its outside surface (5) and in its inside surface (6), which notches are offset angularly so as to enable said outer casing (2) to expand diametrically.

24. A device according to anyone of preceding claims, wherein the outer casing (2) is made of a biocompatible deformable material, e.g. metal or polyethylene.

25. A surgical kit for treating the pathology of flat feet, said surgical kit comprising:
a surgical device (1) according to anyone of claims 1 to 24, including graspable means (40); and
a fitting instrument (50) suitable for being connected mechanically to the graspable means (40) and for exerting a traction force on said graspable means, while also pushing the expansion member (3) away in the compression direction (F) so that said expansion member penetrates progressively into the outer casing (2) while causing said outer casing to expand.
